# EUROPÄISCHE PATENTSCHRIFT

(11) **EP 0 500 755 B1**
(45) Veröffentlichungstag und Bekanntmachung des Hinweises auf die Patenterteilung: **15.05.1996**
(21) Anmeldenummer: 90917650.5
(22) Anmeldetag: 07.11.1990
(51) Int. Cl.: C07D 401/04, A01N 43/54, A01N 43/66

(54) **PYRIDIN-DERIVATE, VERFAHREN ZU IHRER HERSTELLUNG, SIE ENTHALTENDE MITTEL UND IHRE VERWENDUNG ALS FUNGIZIDE**
PYRIDINE DERIVATIVES, PROCESS FOR MAKING THEM, AGENTS CONTAINING THEM AND THEIR USE AS FUNGICIDES
DERIVES DE LA PYRIDINE, LEUR PROCEDE DE FABRICATION, PRODUITS LES RENFERMANT ET LEUR UTILISATION COMME FONGICIDES

(30) Priorität: 09.11.1989 DE 3937285
(43) Veröffentlichungstag der Anmeldung: 02.09.1992
(73) Patentinhaber: Hoechst Schering AgrEvo GmbH, D-13342 Berlin (DE)
(72) Erfinder: GIENCKE, Wolfgang, D-6238 Hofheim am Taunus (DE); SACHSE, Burkhard, D-6233 Kelkheim (DE); WICKE, Heinrich, D-6239 Eppstein (DE)
(86) Internationale Anmeldenummer: EP9001857
(87) Internationale Veröffentlichungsnummer: WO9107399

(56) Entgegenhaltungen:
- EP-A- 0 234 104
- EP-A- 0 259 139

## Beschreibung

Pyridin-Derivate, Verfahren zu ihrer Herstellung, sie enthaltende Mittel und ihre Verwendung als Fungizide

Die vorliegende Erfindung betrifft neue Pyridin-Derivate, Verfahren zu ihrer Herstellung, sie enthaltende Mittel und ihre Verwendung als Fungizide.

Pyridin-Derivate sind bereits als wirksame Komponenten in fungiziden Mitteln bekannt (vgl. EP-A-270 362, EP-A 278 610, EP-A-259 139, EP-A 234 104). Die Wirkung dieser Verbindungen ist jedoch insbesondere bei niedrigen Aufwandmengen nicht immer befriedigend.

Es wurden neue Pyridin-Derivate gefunden, die vorteilhafte Wirkungen bei der Bekämpfung eines breiten Spektrums phytopathogener Pilze insbesondere bei niedrigen Dosierungen aufweisen.

Gegenstand der vorliegenden Erfindung sind daher die Verbindungen der Formel I, 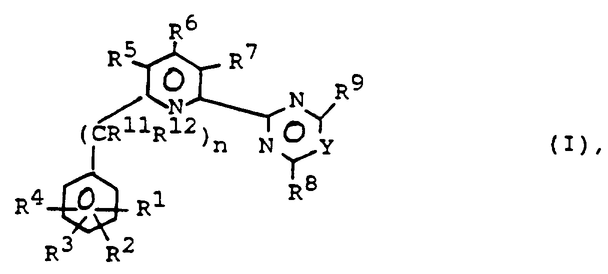 worin
- R¹, R, R³, R⁴ =: unabhängig voneinander Wasserstoff, Halogen, Nitro, (C₁-C₄)Alkyl, (C₁-C₄)Alkoxy, (C₁-C₄)Alkylthio, (C₁-C₄)Haloalkoxy oder (C₁-C₄)Haloalkyl,
- R⁵, R⁶, R⁷ =: unabhängig voneinander Wasserstoff, (C₁-C₆)Alkyl oder Phenyl, wobei der Phenylrest bis zu dreifach durch Halogen, Nitro, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
- R⁸ und R⁹ =: unabhängig voneinander Wasserstoff, (C₁-C₆)Alkyl, (C₃-C₇)Cycloalkyl, (C₃-C₇)Cycloalkyl-(C₁-C₄)alkyl, wobei die beiden letztgenannten Reste im Cycloalkylteil bis zu dreifach durch (C₁-C₄)Alkyl substituiert sein können, (C₁-C₄)Haloalkyl, (C₁-C₄)Alkoxy, (C₁-C₄)Alkylthio, (C₁-C₄)Alkoxy-(C₁-C₄)alkyl, (C₁-C₄)Alkylthio-(C₁-C₄)alkyl, Halogen, (C₂-C₆)Alkenyl, (C₂-C₆)Alkinyl, Phenyl, Phenoxy, Phenoxy-(C₁-C₄)alkyl, Phenylmercapto-(C₁-C₄)alkyl, Phenylmercapto, Phenyl-(C₁-C₄)alkoxy oder Phenyl-(C₁-C₄)alkylthio, wobei die sieben letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, oder (C₂-C₄)Alkenyloxy, (C₂-C₄)Alkinyloxy, (C₁-C₄)Haloalkoxy, (C₁-C₄)Alkoxy- (C₁-C₄)alkoxy, (C₁-C₄)Alkylthio-(C₁-C₄)alkylthio,
- R¹⁰ =: Wasserstoff, (C₁-C₄)Alky), (C₁-C₄)Alkoxy, (C₂-C₆)-Alkenyloxy, (C₂-C₆)Alkinyloxy, (C₁-C₄)Alkylthio, Halogen, Phenyl, wobei der Phenylrest bis zu dreifach durch Halogen, Nitro, Cyano, (C₁-C₄)Alkyl, (C₁-C₄)-Alkoxy, (C₁-C₄)Alkylthio, (C₁-C₄)Haloalkyl oder (C₁-C₄)Haloalkoxy substituiert sein kann, oder
- R⁸ und R¹⁰: bilden zusammen eine Polymethylenkette der Formel -(CH₂)ₘ- mit m = 3 - 4,
- R¹¹ und R¹ =: unabhängig voneinander Wasserstoff oder (C₁-C₄)Alkyl,
- Y =: N oder -CR¹⁰ und
- n =: 1-3 bedeuten, sowie deren Säureadditionssalze.

Dabei können die Alkyl-, Alkenyl- oder Alkinylreste sowohl geradkettig als auch verzweigt sein. Halogen bedeutet F, Cl, Br, J, bevorzugt F, Cl und Br. Die Vorsilbe "Halo" in der Bezeichnung eines Substituenten bedeutet hier und im folgenden, daß dieser Substituent einfach oder mehrfach bei gleicher oder verschiedener Bedeutung auftreten kann. Die Vorsilbe "Halo" beinhaltet Fluor, Chlor, Brom oder Jod, insbesondere Fluor, Chlor oder Brom. Als Beispiele für Halogenalkyl seien genannt: CF₃, CF₂CHF₂, CF₂CF₃, CCl₃, CCl₂F, CF₂CF₂CF₃, CF₂CHFCF₃ und (CF₂)₃CF₃. Beispiele für Haloalkoxy sind OCF₃, OCF₂CHF₂ oder OCF₂CF₂CF₃.

Bevorzugt unter den Verbindungen der Formel I sind solche, worin
- R¹, R, R³, R⁴ =: unabhängig voneinander Wasserstoff, Halogen, (C₁-C₂)Haloalkyl oder (C₁-C₃)Alkyl,
- R⁵, R⁶ =: unabhängig voneinander Wasserstoff, (C₁-C₃)Alkyl oder Phenyl, wobei der Phenylrest bis zu dreifach durch Halogen oder (C₁-C₄)Alkyl substituiert sein kann,
- R⁷ =: Wasserstoff,
- R⁸ und R⁹: unabhängig voneinander Wasserstoff, (C₁-C₆)Alkyl, (C₃-C₆)Cycloalkyl, (C₅-C₆)Cycloalkyl-(C₁-C₃)alkyl, Halogen, Phenyl, das bis zu dreifach durch Halogen, (C₁-C₄)Alkyl oder (C₁-C₄)Alkoxy substituiert sein kann, oder (C₂-C₄)Alkenyloxy, (C₂-C₄)Alkinyloxy, (C₁-C₄)Haloalkoxy oder (C₁-C₄)Alkoxy-(C₁-C₄)alkoxy,
- R¹⁰ =: Wasserstoff, (C₁-C₄)Alkyl, Halogen, Phenyl, wobei der Phenylrest bis zu dreifach durch Halogen oder (C₁-C₃)Alkyl substituiert sein kann, oder (C₁-C₃)Alkoxy oder
- R⁸ und R¹⁰: bilden zusammen eine Polymethylenkette der Formel -(CH₂)ₘ- mit m = 3 - 4,
- R¹¹, R¹ =: Wasserstoff,
- Y =: N oder -CR¹⁰
- und n = 1: bedeuten, sowie deren Säureadditionssalze.

Zur Herstellung der Säureadditionssalze der Verbindungen der Formel I kommen folgende Säuren in Frage: Halogenwasserstoffsäuren wie Chlorwasserstoffsäure oder Bromwasserstoffsäure, ferner Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionssalze der Verbindungen der Formel I können in einfacher Weise nach üblichen Salzbildungsmethoden, z. B. durch Lösen einer Verbindung der Formel I in einem geeigneten organischen Lösemittel und Hinzufügen der Säure erhalten werden und in bekannter Weise, z. B. durch Abfiltrieren, isoliert und gegebenenfalls durch Waschen mit einem inerten organischen Lösemittel gereinigt werden.

Gegenstand der vorliegenden Erfindung ist auch ein Verfahren zur Herstellung der Verbindungen der Formel I.

Diese können nach den folgenden Varianten dargestellt werden:
1) Pyridin-Derivate der Formel I mit R⁹ = H und Y = -CR¹⁰ lassen sich durch reduktive Dehalogenierung von entsprechenden Halopyrimidinen der Formel I, worin R⁹ = Halogen (z.B. Cl, Br, J) bedeutet und die restlichen Substituenten wie in Formel I definiert sind, erhalten.
   Die Dehalogenierung kann mit Wasserstoff in Gegenwart von Katalysatoren (z.B. Palladium/Kohle) in einem inerten Lösungsmittel, z.B. Wasser, niederer Alkohol (wie Methanol und Ethanol), Ethylacetat oder Toluol oder Gemischen derselben durchgeführt werden. Vorteilhaft ist die Zugabe von Basen wie Alkali- oder Erdalkalihydroxide bzw. -carbonate. Die Reaktion wird im Bereich von 15-60 °C unter einem Druck von 1 bis 5 bar durchgeführt.
2) Pyridin-Derivate der Formel I, worin R⁹ für (C₁-C₄)Alkoxy, (C₁-C₄)Alkylthio, Phenoxy, Phenylmercapto, Phenyl-(C₁-C₄)alkoxy oder Phenyl-(C₁-C₄)alkylthio, wobei die 4 letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, oder (C₂-C₄)Alkenyloxy (C₂-C₄)Alkinyloxy, (C₁-C₄)Haloalkoxy, (C₁-C₄)Alkoxy-(C₁-C₄)alkoxy
   oder (C₁-C₄)Alkylthio-(C₁-C₄)alkylthio steht und Y = -CR¹⁰ bedeutet, können durch Reaktion der entsprechenden Halopyrimidine I (R⁹ = Halogen) mit einer Alkalimetallverbindung der Formel II R⁹M (II), worin R⁹ die obengenannte Bedeutung besitzt und M für ein Alkalimetall (z.B. Li, Na, K) steht, hergestellt werden.
   Die Reaktion kann zwischen 0 °C und 130 °C innerhalb von 0,5 h bis 72 h durchgeführt werden. Die Alkalimetallverbindung (II) kann in Mengen von 1 bis 2 Moläquivalenten bezogen auf 1 Äquivalent des Halopyrimidins (I) (R⁹ = Halogen) eingesetzt werden. Die Reaktion wird gewöhnlich in Gegenwart eines Lösungsmittels durchgeführt.
   In den Fällen, in denen eine Alkalimetallverbindung R⁹M eingesetzt wird, worin R⁹ für (C₁-C₄)Alkoxy, Phenyl- (C₁-C₄)alkoxy, (C₂-C₄)Alkenyloxy, (C₂-C₄)-Alkinyloxy, (C₁-C₄)Haloalkoxy oder (C₁-C₄)Alkoxy-(C₁-C₄)alkoxy steht, wird der korrespondierende Alkohol R⁹OH oder ein Ether (z.B. Diethylether, Dioxan oder Tetrahydrofuran) oder eine Mischung derselben als Lösungsmittel benutzt.
   In den Fällen, in denen eine Alkalimetallverbindung eingesetzt wird, worin R⁹ für (C₁-C₄)Alkylthio, Phenoxy, Phenylmercapto, Phenyl-(C₁-C₄)alkoxy oder Phenyl-(C₁-C₄)alkylthio steht, wird ein Ether (z.B. Diethylether, Dioxan oder Tetrahydrofuran), ein Nitril (z.B. Acetonitril), ein aromatischer Kohlenwasserstoff (z.B. Toluol oder Xylol) oder eine Mischung derselben als Lösungsmittel verwendet.
3) Pyridin-Derivate der Formel I, worin R⁹ für (C₁-C₆)Alkyl, (C₃-C₇)Cycloalkyl, (C₃-C₇)Cycloalkyl-(C₁-C₄)alkyl, (C₁-C₄)Alkoxy-(C₁-C₄)alkyl, (C₁-C₄)Alkylthio-(C₁-C₄)alkyl oder Phenyl steht, wobei der letztgenannte Rest bis zu dreifach durch (C₁-C₄)Alkyl oder (C₁-C₄)Alkoxy substituiert sein kann, und Y = -CR¹⁰ bedeutet, können durch Umsetzung von entsprechenden Halopyrimidinen I (R⁹ = Halogen) mit Grignard-Verbindungen R⁹MgX (III), wobei R⁹ wie oben angegeben definiert ist und X für Halogen (Cl, Br, J) steht, in Gegenwart von Nickel-Phosphin-Komplexen wie z.B. 1,2-Bis-(diphenylphosphino)-ethan-nickel-(II)-chlorid oder 1,3-Bis-(diphenylphosphino)-propan-nickel-(II)-chlorid erhalten werden (vgl. Chem. Pharm. Bull. 26, 2160 (1978)).
   Die Reaktion kann zwischen 0 °C und 80 °C bzw. bei dem Siedepunkt des Lösungsmittels innerhalb von 2-48 h durchgeführt werden. Die Grignard-Verbindung R⁹MgX wird dabei in Mengen von 1-2,5 Moläquivalenten bezogen auf 1 Äquivalent Halopyrimidin (I) eingesetzt. Als Lösungsmittel eignen sich Ether wie z.B. Diethylether, THF, Dioxan, Dimethoxyethan.
   Die Halopyrimidine I (R⁹ = Halogen) können durch Umsetzung der entsprechenden Hydroxypyrimidine (I), worin R⁹ für den Rest OH steht und die übrigen Substituenten die Bedeutungen wie in Formel I besitzen, mit Halogenierungsagens erhalten werden. Als Halogenierungsagens können z.B. Thionylchlorid, Phosgen, Phosphoroxychlorid, Phosphorpentachlorid, Phosphoroxybromid oder Phosphortribromid eingesetzt werden. Die Reaktionen können in einem Lösungsmittel, aber auch ohne Lösungsmittel durchgeführt werden. Das Halogenierungsagens wird in Mengen von 1 bis 4 Äquivalenten bezogen auf 1 Äquivalent des Hydroxypyrimidins (I) (R⁹ = OH) eingesetzt. Die Reaktionen können in einem Temperaturbereich von 25 - 160 °C durchgeführt werden. Als Lösungsmittel dienen z.B. aromatische Kohlenwasserstoffe (z.B. Benzol oder Toluol, u.a.) oder halogenierte Kohlenwasserstoffe (z.B. Chlorbenzol).
   Die Hydroxypyrimidine (I) (R⁹ = OH) können durch Kondensation der Amidin-Derivate (IV) mit β-Oxocarboxylaten V dargestellt werden, 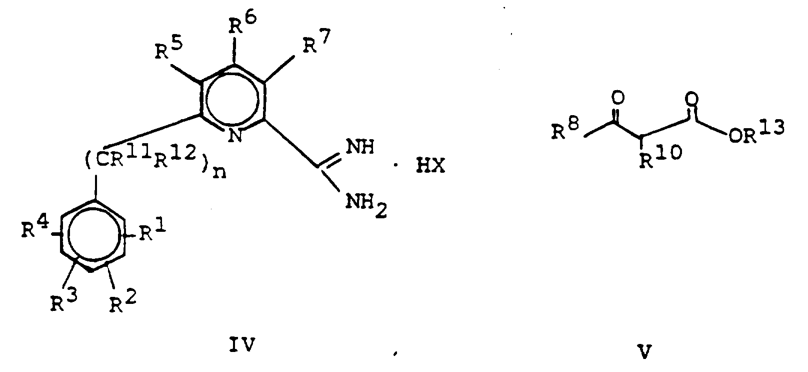 worin R¹-R⁸, R¹⁰-R¹ und n wie in Formel I definiert sind, X für Halogen (z.B. Chlor, Brom, Jod) und R¹³ für niedere Alkylreste wie Methyl, Ethyl und Propyl steht.
   Die Reaktionen werden im Temperaturbereich von 20 - 110 °C innerhalb von 2 - 72 h durchgeführt. Das β-Oxocarboxylat (V) kann in Mengen von 1 - 1,5 Äquivalenten bezogen auf 1 Äquivalent Amidin-Derivat (IV) eingesetzt werden. Die Reaktion wird in Gegenwart einer Base und eines Lösungsmittels durchgeführt. Als Basen können z.B. anorganische Basen wie Alkalimetallhydroxide und -carbonate oder organische Basen wie Natriumalkoxide, Trialkylamine und N,N-Dialkylaniline verwendet werden. Als Lösungsmittel finden niedere Alkohole (wie Methanol und Ethanol), cyclische Ether (wie Dioxan und THF), Pyridin, N,N-Dimethylformamid, Wasser oder Gemische derselben Verwendung. Die Amidin-Derivate (IV) und die β-Oxocarboxylate lassen sich nach an sich bekannten Verfahren herstellen (vgl. J. Org. Chem. 32, 1591 (1967) bzw. Synthesis 1982, 451 und Organikum 1986, 516ff).
4) Pyridin-Derivate der Formel (I), worin R⁸ = R⁹ für (C₁-C₄)Alkyl, Phenyl-(C₁-C₄)alkyl oder Phenyl steht, wobei die beiden letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, und Y = N bedeutet, lassen sich durch Umsetzung der Amidin-Derivate IV mit einem Imidat-Derivat der Formel VI herstellen, worin R⁹ 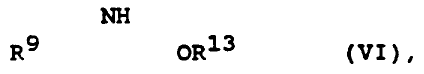 die obengenannte Bedeutung hat und R¹³ wie in Formel V definiert ist (vgl. auch JOC 27, 3608 (1962)).
   Die Reaktion kann in Gegenwart eines Lösungsmittels oder auch ohne Lösungsmittel durchgeführt werden. Als Lösungsmittel eignen sich niedere Alkohole wie Methanol und Ethanol. Die Umsetzung erfolgt im Temperaturbereich von 10 °C bis 100 °C. Das Imidat-Derivat (VI) kann in Mengen von 2 - 5 Äquivalenten bezogen auf 1 Äquivalent Amidin-Derivat IV eingesetzt werden.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze, wie z.B. Piricularia oryzae, Venturia inaequalis, Cercospora beticola, Echte Mehltauarten, Fusariumarten, Plasmopora viticola, Pseudoperonospora cubensis, Leptosphaeria nodorum, Drechslera, verschiedene Rostpilze und Pseudocercosporella herpotrichoides. Besonders gut werden Benzimidazol- und Dicarboximid-sensible und -resistente Botrytis cinerea Stämme erfaßt.

Die erfindungsgemäßen Verbindungen eignen sich daneben auch für den Einsatz in technischen Bereichen, beispielsweise als Holzschutzmittel, als Konservierungsmittel in Anstrichfarben, in Kühl schmiermitteln für die Metallbearbeitung oder als Konservierungsmittel in Bohr- und Schneidölen.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage:

Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C-Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierugen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z.B. folgende Produkte zu nennen: Imazalil, Prochloraz, Fenapanil, SSF 105, Triflumizol, PP 969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol,
Bupirimate, Rabenzazole, Tricyclazole, Fluobenzimine, Pyroxyfur, NK-483, PP-389, Pyroquilon, Hymexazole, Fenitropan, UHF-8227, Cymoxanil, Dichlorfluanid, Captafol, Captan, Folpet, Tolylfluanid, Chlorothalonil, Etridiazol, Iprodione (Formel II), Procymidon, Vinclozolin, Metomeclan, Myclozolin, Dichlozolinate, Fluorimide, Drazoxolan, Chinomethionate, Nitrothalisopropyl, Dithianon, Dinocap, Binapacryl, Fentinacetate, Fentinhydroxide, Carboxin, Oxycarboxin, Pyracarbolid, Methfuroxam, Fenfuram, Furmecyclox, Benodanil, Mebenil, Mepronil, Flutalanil, Fuberidazole, Thiabendazole, Carbendazim, Benomyl, Thiophanate, Thiophanatemethyl, CGD-94340 F, IKF-1216,
Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosethylaluminium,
Natrium-dodecylbenzolsulfonat,
Natrium-dodecylsulfat,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,
Cetyl-trimethyl-ammoniumchlorid,

Salze von langkettigen primären, sekundären oder tertiären Aminen, Alkyl-propylenamine, Lauryl-pyridinium-bromid, ethoxilierte quaternierte Fettamine, Alkyl-dimethyl-benzyl-ammoniumchlorid und 1 Hydroxyethyl-2-alkyl-imidazolin.

Die oben genannten Kombinationspartner stellen bekannte Wirkstoffe dar, die zum großen Teil in C.R. Worthing, S.B. Walker, The Pesticide Manual, 7. Auflage (1983), British Crop Protection Council beschrieben sind.

Darüberhinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen, hergestellte Stoffe u.a.. Bevorzugte Mischungspartner sind:
1. aus der Gruppe der Phosphorsäureester Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorphon.
2. aus der Gruppe der Carbamate Aldicarb, Bendiocarb, BPMC (2-(1-Methylpropyl)phenyl-methylcarbamat), Butocarboxim, Butoxycarboxim, Carbaryl, Carbofuran, Carbosulfan, Cloethocarb, Isoprocarb, Methomyl, Oxamyl, Pirimicarb, Promecarb, Propoxur, Thiodicarb.
3. aus der Gruppe der Carbonsäureester Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethyl-vinyl)cyclopropancarbonsäure-(alpha-cyano-3-phenyl-2-methyl-benzyl)ester (FMC 54800), Fenpropathrin, Fenfluthrin, Fenvalerat, Flucythrinate, Flumethrin, Fluvalinate, Permethrin, Resmethrin, Tralomethrin.
4. aus der Gruppe der Formamidine Amitraz, Chlordimeform
5. aus der Gruppe der Zinnverbindungen Azocyclotin, Cyhexatin, Fenbutatinoxid
6. Sonstige
   Abamektin, Bacillus thuringiensis, Bensultap, Binapacryl, Bromopropylate, Buprofecin, Camphechlor, Cartap, Chlorbenzilate, Chlorfluazuron, 2-(4-Chlorphenyl)-4,5-diphenylthiophen (UBI-T 930), Chlofentezine, Cyclopropancarbonsäure(2-naphthylmethyl)ester (Ro 12-0470), Cyromacin, DDT, Dicofol, N-(3,5-Dichlor-4-(1,1,2,2-tetrafluoroethoxy)phenylamino)carbonyl)-2,6-difluorbenzamide (XRD 473), Diflubenzuron, N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Flufenoxuron, Gamma-HCH, Hexythiazox, Hydramethylnon (AC 217 300) Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinon-3-yl-carbamaldehyde (WL 108 477), Propargite, Teflubenzuron, Tetradifon, Tetrasul, Thiocyclam, Triflumaron, Kernpolyeder- und Granuloseviren.

Der Wirkstoffgehalt der aus den handelsüblichen Formulierungen bereiteten Anwendungsformen kann in weiten Bereichen variieren, Die Wirkstoffkonzentration der Anwendungsformen kann von 0,0001 bis zu 100 Gew.-% Wirkstoff, vorzugsweise zwischen 0,001 und 1 Gew.-% liegen. Die Anwendung geschieht in einer den Anwendungsformen angepaßten üblichen Weise.

Nachfolgende Beispiele dienen zur Erläuterung der Erfindung.

### A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### B. Chemische Beispiele

### 2-(6-Benzyl-pyridin-2-yl)-4-propyl-pyrimidin (Beispiel-Nr.: 1.2)

Zu einer Lösung von 3,24 g (0,01 mol) 2-(6-Benzyl-pyridin-2-yl)-6-chlor-4-propyl-pyrimidin in 50 ml Ethanol fügt man 0,2 g 5 % Palladium/Kohle. Unter einem Druck von 3 bar und bei einer Temperatur von 60 °C bringt man dieses Gemisch unter starkem Rühren 2 h in Kontakt mit Wasserstoff.

Anschließend wird der Katalysator abfiltriert und das Filtrat im Vakuum eingeengt. Der Rückstand wird in Wasser aufgenommen, mit Natriumbicarbonat gesättigt und mit CH₂Cl₂ extrahiert. Die organische Phase wird über Na₂SO₄ getrocknet und eingeengt. Man erhält 2,85 g (98,6 %) eines gelblichen Öls.

### 2-(6-Benzyl-pyridin-2-yl)-4-methoxy-6-phenyl-pyrimidin (Beispiel-Nr.: 1.15)

Eine Natriummethylat-Lösung wird durch Auflösen von 0,184 g (0,008 mol) Natrium in 40 ml abs. Methanol hergestellt. Zu dieser Lösung fügt man 1,79 g (0,005 mol) 2-(6-Benzyl-pyridin-2-yl)-4-chlor-6-phenyl-pyrimdin und kocht 3 h am Rückfluß. Danach wird eingeengt, der Rückstand mit Wasser versetzt und mit Methylenchlorid extrahiert. Die organische Phase wird mit Na₂SO₄ getrocknet und eingeengt. Nach der Kristallisation aus Diisopropylether erhält man 1,49 g (84,3 %) eines farblosen Feststoffes.
Schmp.: 128 - 129 °C

### 2-(6-Benzyl-pyridin-2-yl)-4,6-dimethyl-1,3,5-triazin (Beispiel-Nr. 2.1)

2,47 g (0,01 mol) 6-Benzyl-2-picolinamidin-Hydrochlorid werden zusammen mit 3,48 g (0,04 mol) Ethylacetimidat 2 h bei 60 °C gerührt. Die Reaktionsmischung wird danach in Wasser aufgenommen und mit CHCl₃ extrahiert. Die organische Phase wird mit Wasser gewaschen, über Na₂SO₄ getrocknet und eingeengt. Der Rückstand wird einer Säulenchromatographie (Laufmittel: Methylenchlorid) unterworfen. Man erhält 1,62 g (58,7 %) eines farblosen Öls.

Analog zu diesen Beispielen lassen sich die Verbindungen der Tabelle A herstellen.

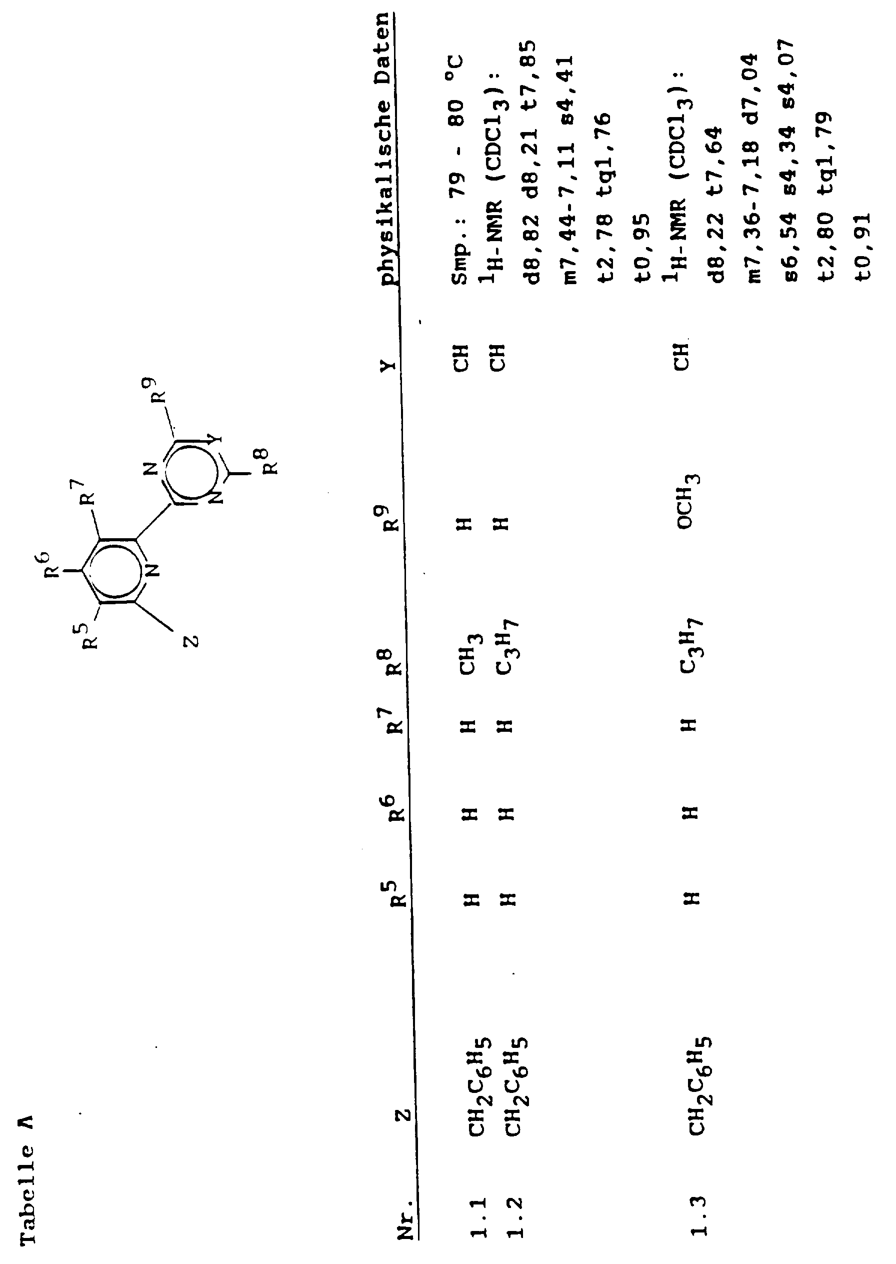

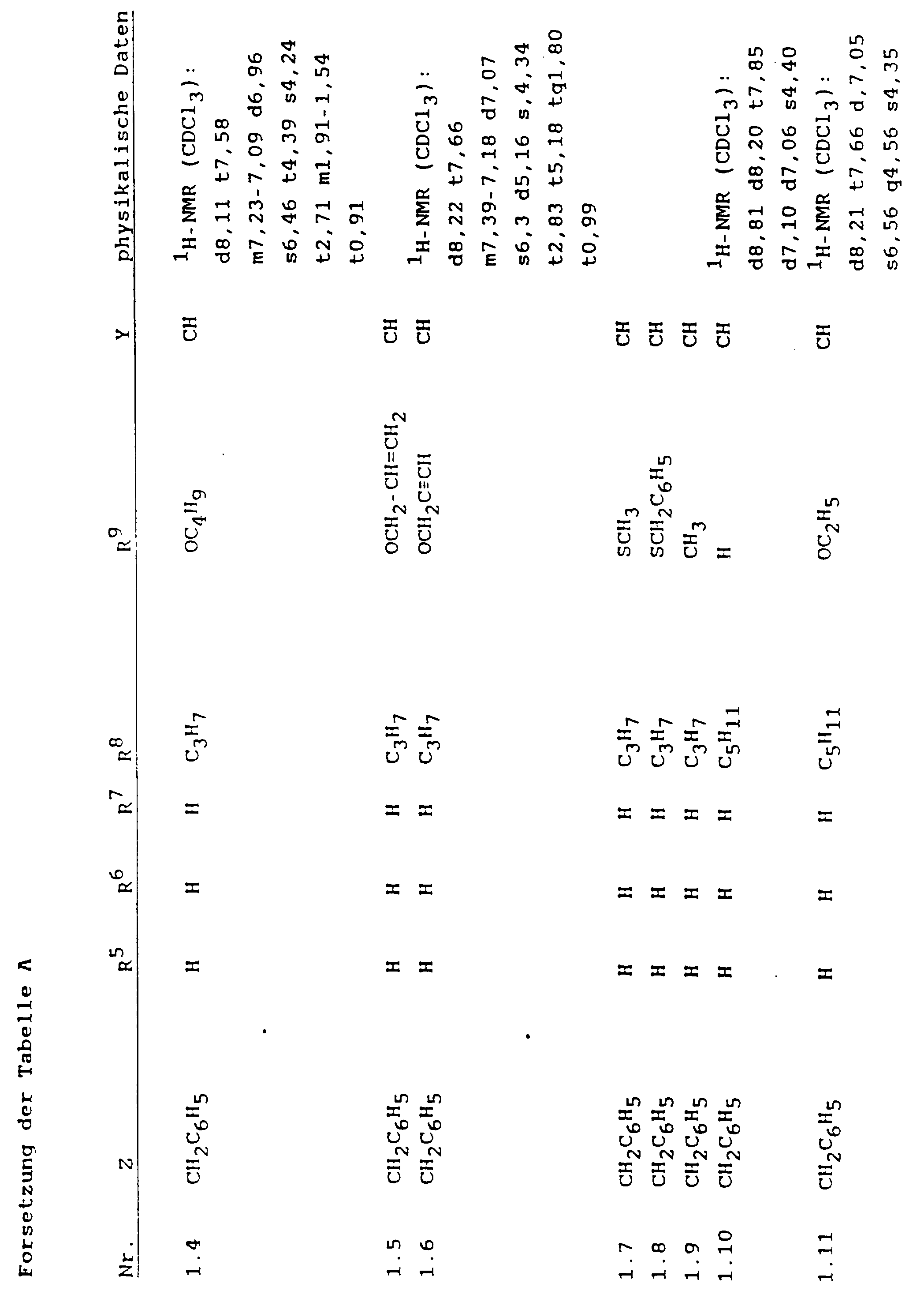

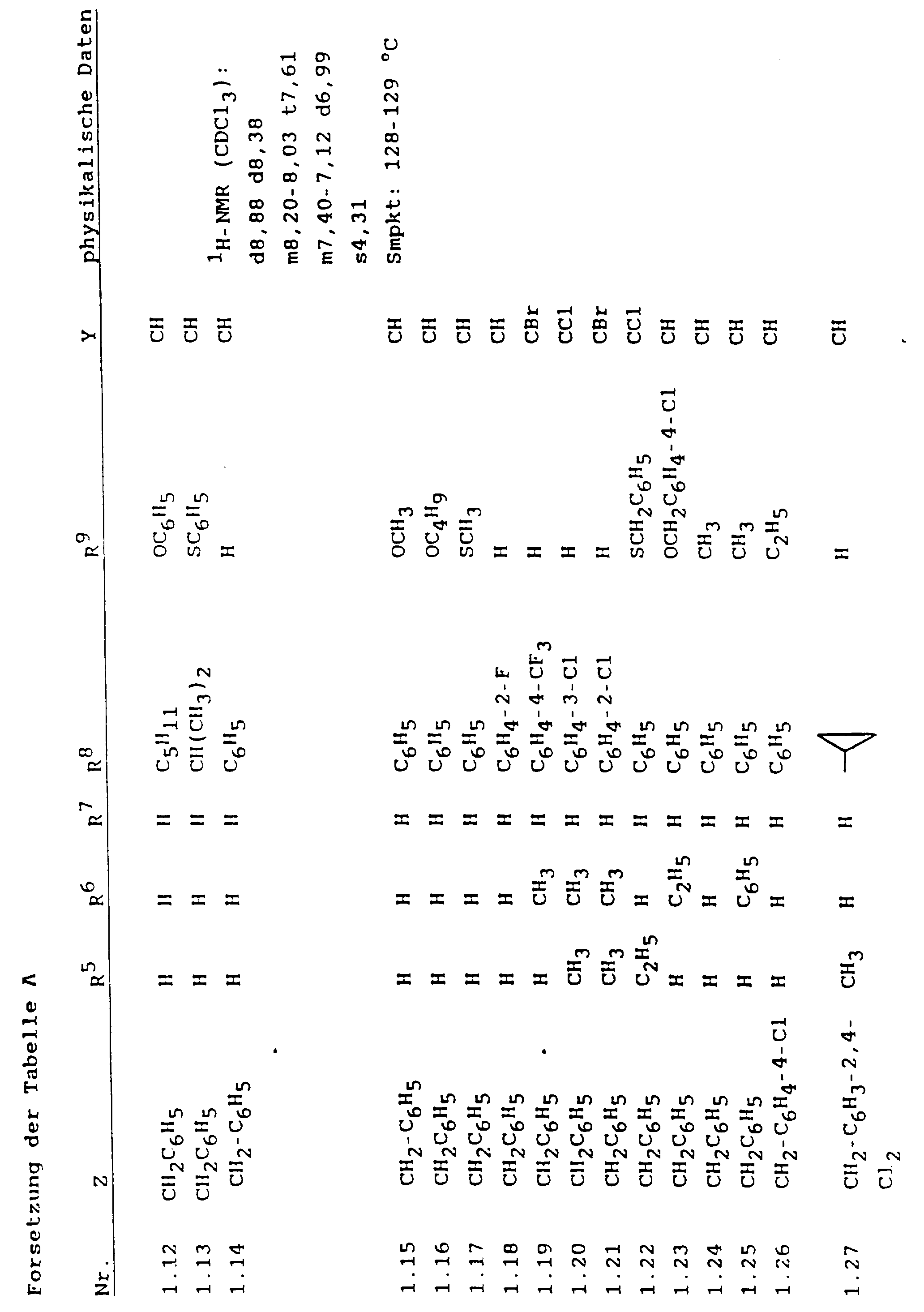

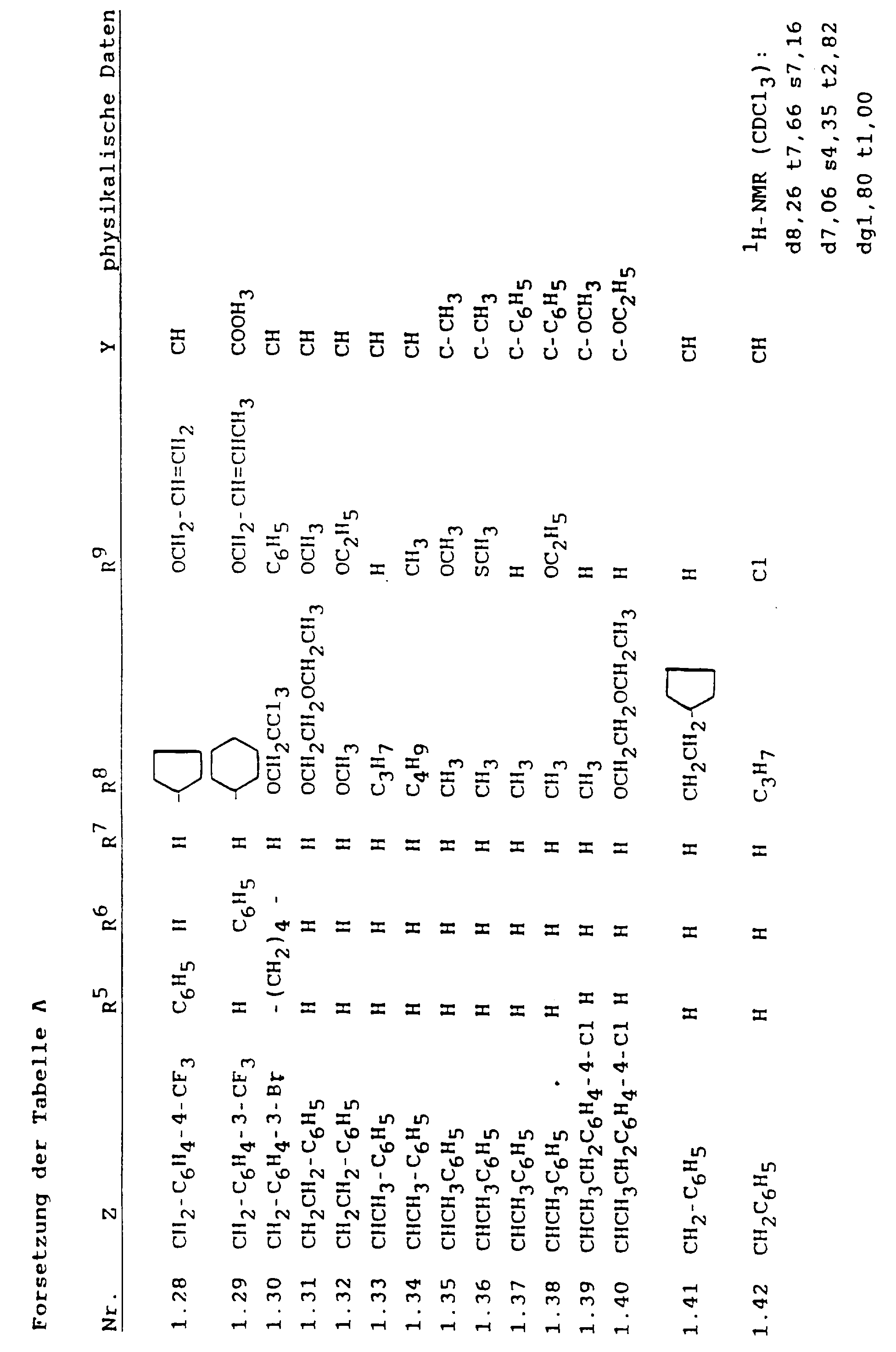

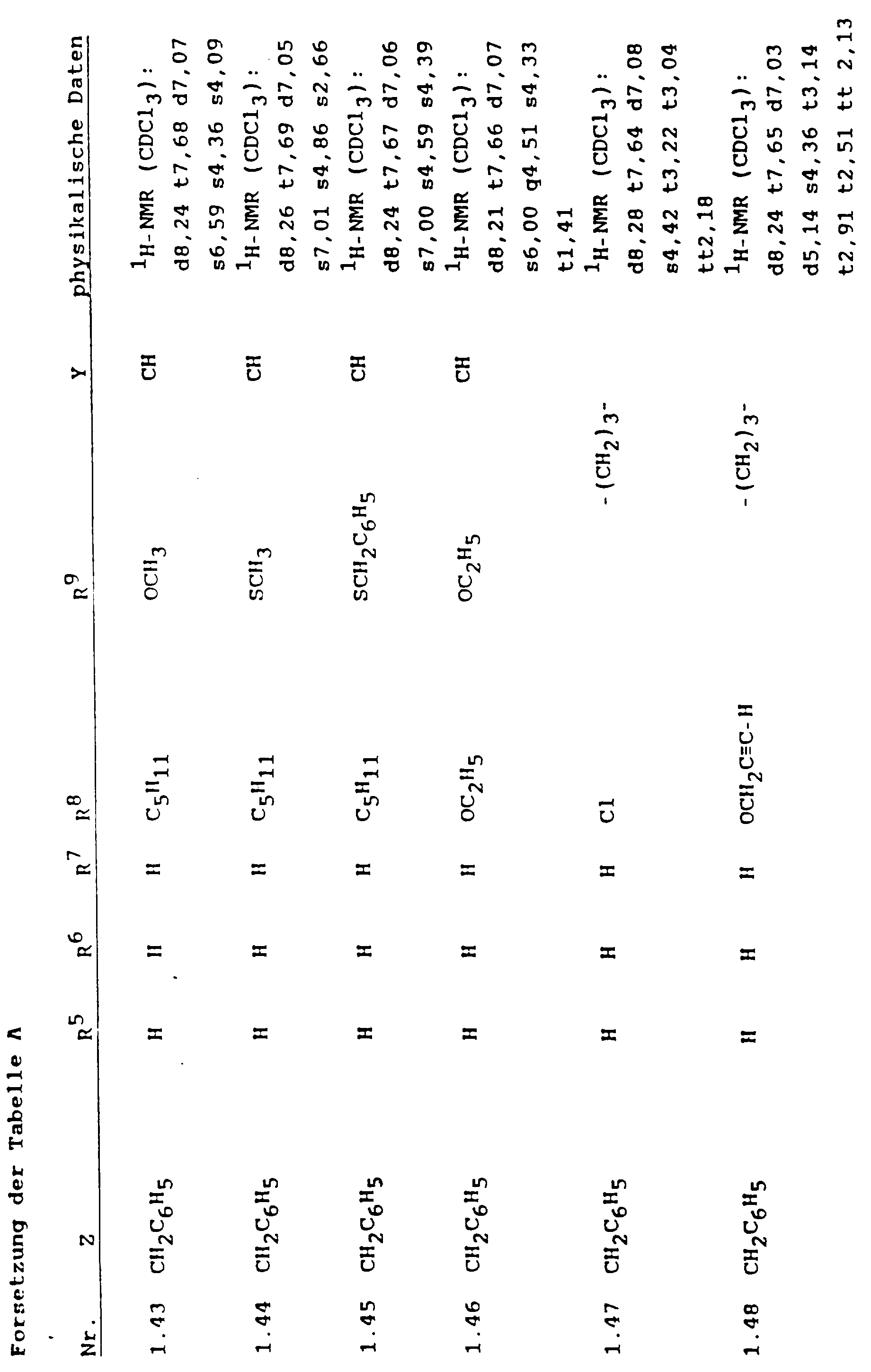

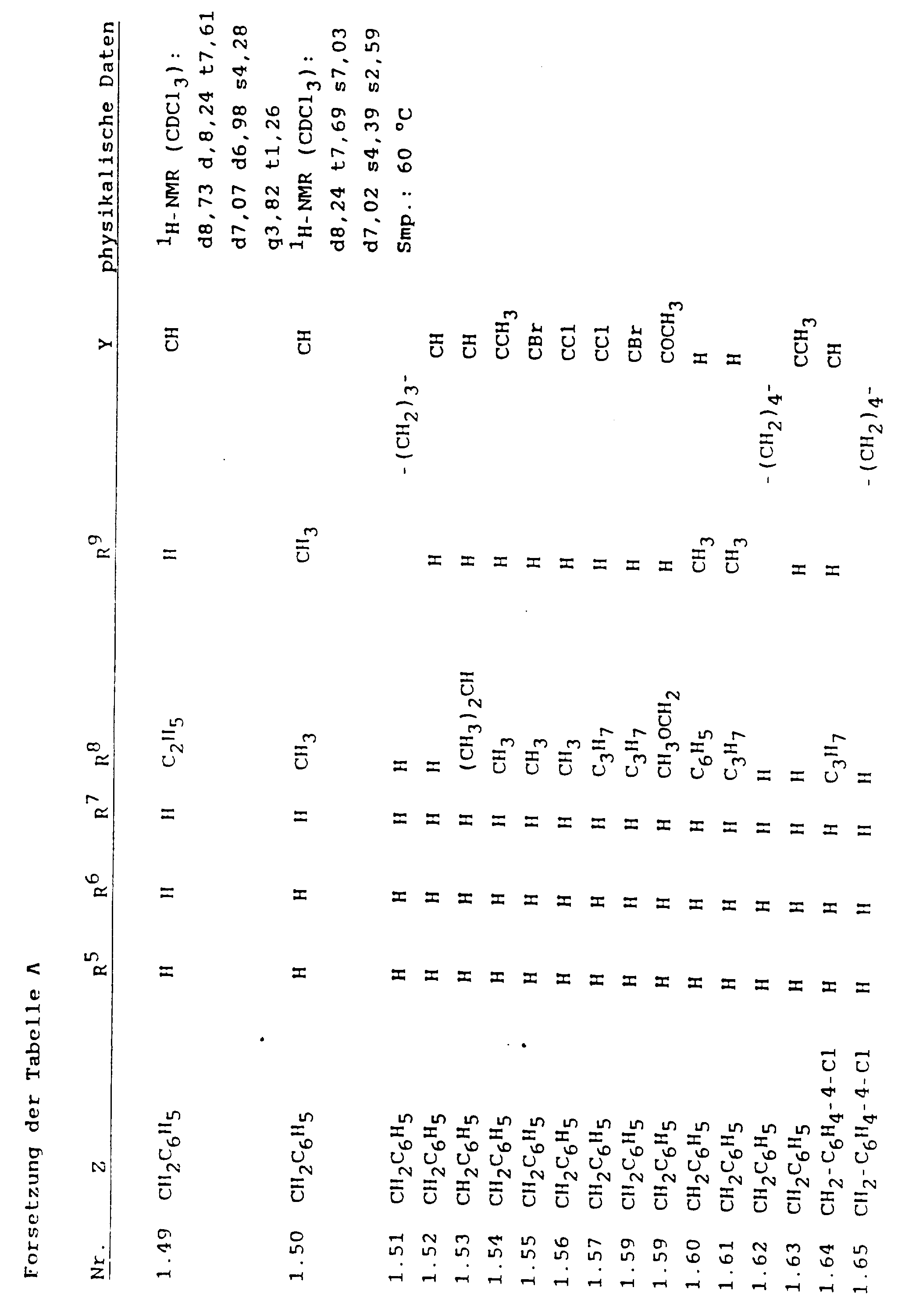

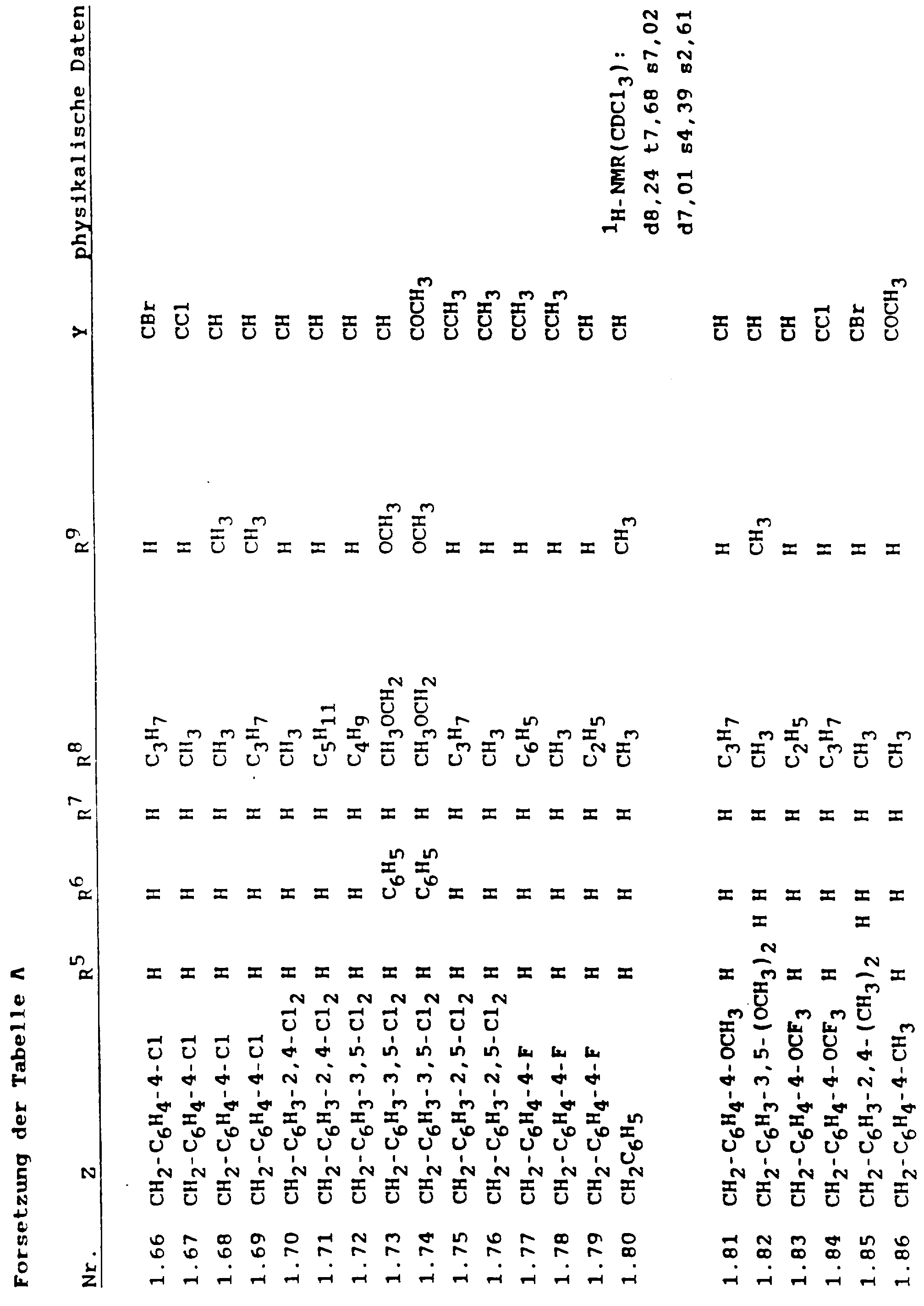

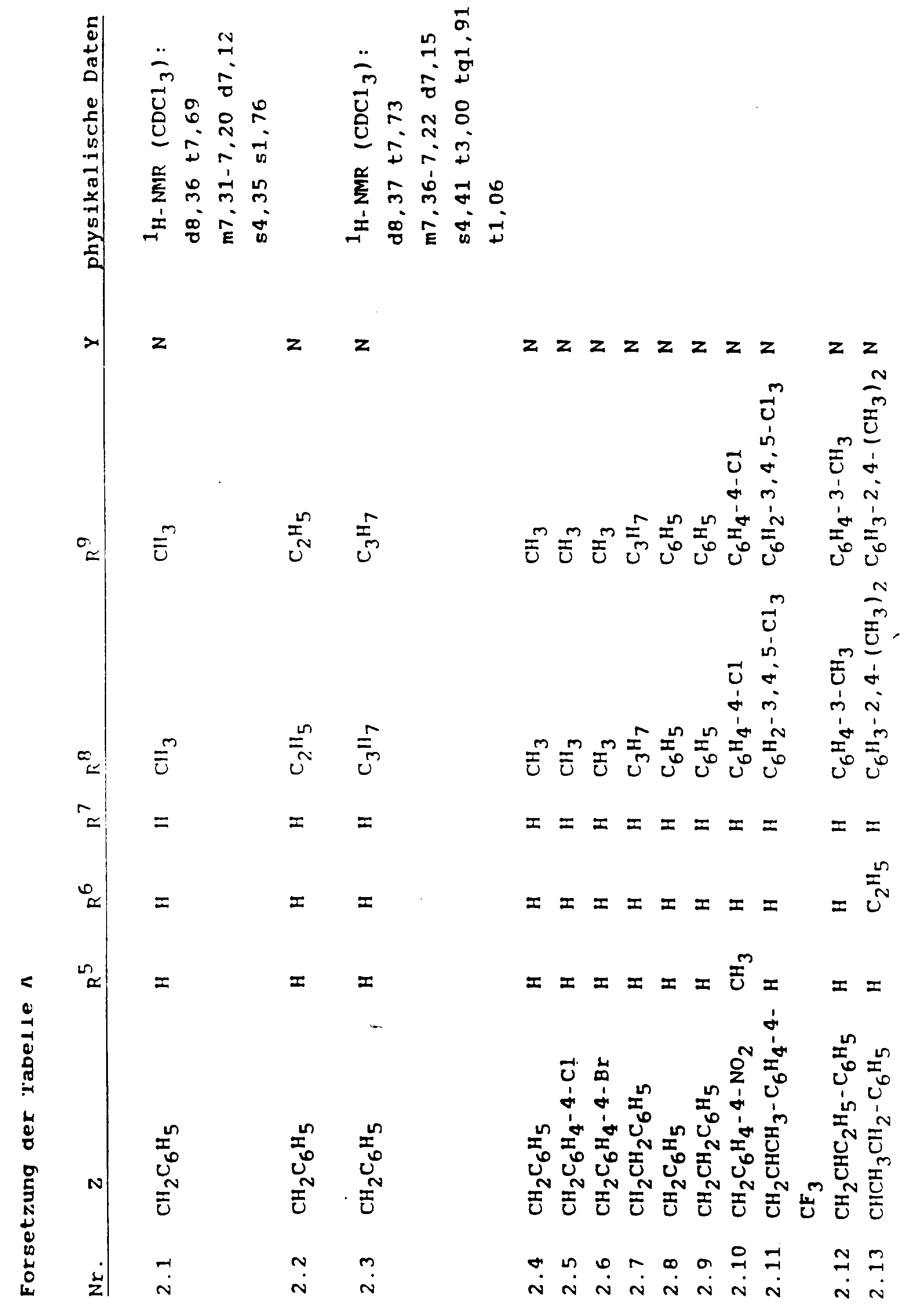

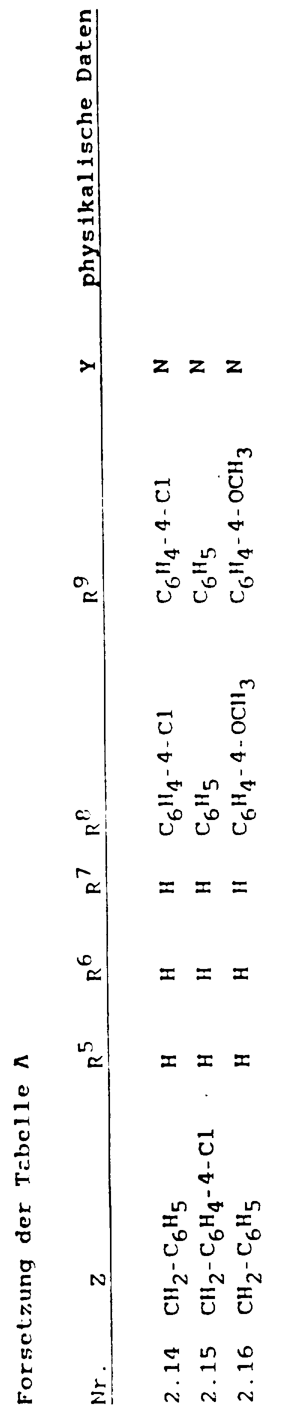

### C. Biologische Beispiele

### Beispiel 1

Etwa 5 Wochen alte Reispflanzen der Sorte "Ballila" wurden nach Vorspritzen mit 0,05 %iger Gelatinelösung mit der unten angegebenen Konzentration der beanspruchten Verbindungen behandelt. Nach Antrocknen des Spritzbelages wurden die Pflanzen mit einer Sporensuspension von Piricularia oryzae gleichmäßig inokuliert und 48 h in eine dunkel gehaltene Klimakammer mit einer Temperatur von 25°C und 100 % rel. Luftfeuchte gestellt. Danach wurden die Reispflanzen in einem Gewächshaus mit einer Temperatur von 25°C und 80 % rel. Luftfeuchte weiterkultiviert. Nach 5 Tagen erfolgte die Befallsauswertung. Der Befallsgrad wurde in % befallener Blattfläche im Vergleich zu unbehandelten, infizierten Kontrollpflanzen ausgedrückt. Die Ergebnisse sind in Tabelle 1 zusammengestellt.

**Tabelle 1**

| Verbindung gemäß Beispiel | mit Piricularia oryzae befallene Blattfläche in % bei mg Wirkstoff/Liter Spritzbrühe |
|---|---|
| | 500 |
| 1.3 | 0 |
| 1.2 | 0 |
| 1.5 | 0 |
| 2.1 | 0 |
| 1.43 | 0 |
| 1.11 | 0 |
| 1.44 | 0 |
| 1.10 | 0 |
| 1.46 | 0 |
| | |
| unbehandelte, infizierte Pflanzen | 100 |

### Beispiel 2

Gerstenpflanzen wurden im 2-Blattstadium mit Konidien des Gerstenmehltaus (Erysiphe graminis hordei) stark inokuliert und in einem Gewächshaus bei 20°C und einer relativen Luftfeuchte von ca. 50 % weiterkultiviert. 1 Tag nach Inokulation wurden die Pflanzen mit den in Tabelle 2 aufgeführten Verbindungen in der angegebenen Wirkstoffkonzentration gleichmäßig benetzt. Nach einer Inkubationszeit von 7-9 Tagen wurden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebniss ist in Tabelle 2 zusammengefaßt.

**Tabelle 2**

| Verbindung gemäß Beispiel | mit Gerstenmehltau befallene Blattfläche in % bei mg Wirkstoff/Liter Spritzbrühe |
|---|---|
| | 500 |
| 1.3 | 0 |
| 1.2 | 0 |
| 1.43 | 0 |
| 1.10 | 0 |
| 1.46 | 0 |
| | |
| unbehandelte, infinizierte Pflanzen | 100 |

### Beispiel 3

Weizen der Sorte "Jubilar" wurde im 2-Blattstadium mit wäßrigen Suspensionen der beanspruchten Verbindungen tropfnaß behandelt. Nach dem Antrocknen ds Spritzbelages wurden die Pflanzen mit einer wäßrigen Sporensuspension von Puccinia recondita inokuliert. Die Pflanzen wurden für ca. 16 Stunden tropfnaß in eine Klimakammer mit 20 °C und ca. 100 % rel. Luftfeuchte gestellt. Anschließend wurden die infizierten Pflanzen in einem Gewächshaus bei einer Temperatur von 22-25 °C und 50-70 % rel. Luftfeuchte weiterkultiviert.

Nach einer Inkubationszeit von ca. 2 Wochen sporuliert der Pilz auf der gesamten Blattoberfläche der nicht behandelten Kontrollpflanzen, so daß eine Befallsauswertung der Versuchspflanzen vorgenommen werden kann. Der Befallsgrad wurde in % befallener Blattfläche im Vergleich zu unbehandelten, infizierten Kontrollpflanzen ausgedrückt und ist in Tabelle 3 wiedergegeben.

**Tabelle 3**

| Verbindung gemäß Beispiel | mit Puccinia recondita befallene Blattfläche in % bei mg Wirkstoff/Liter Spritzbrühe |
|---|---|
| | 500 |
| 1.3 | 0 |
| 1.2 | 0 |
| unbehandelte, infizierte Pflanzen | 100 |

### Beispiel 4

Weizenpflanzen der Sorte "Jubilar" wurden im 2-Blattstadium mit wäßrigen Suspensionen der in Tabelle 4 angegebenen Präparate tropfnaß behandelt.

Nach dem Antrocknen des Spritzbelages wurden die Pflanzen mit einer wäßrigen Pyknosporen-Suspension von Leptosphaeria nodorum inokuliert und mehrere Stunden bei 100 % rel. Luftfeuchte in einer Klimakammer inkubiert. Bis zur Symptomausprägung wurden die Pflanzen im Gewächshaus bei ca. 90 % rel. Luftfeuchte weiterkultiviert.

Der Wirkungsgrad ist prozentual zur unbehandelten, infizierten Kontrolle ausgedrückt und wird in Tabelle 4 wiedergegeben.

**Tabelle 4**

| Verbindung gemäß Beispiel | mit Leptosphaeria nodorum befallene Blattfläche in % bei mg Wirkstoff/Liter Spritzbrühe |
|---|---|
| | 500 |
| 1.43 | 0 |
| 1.11 | 0 |
| 1.44 | 0 |
| 1.46 | 0 |
| 1.14 | 0 |
| 1.5 | 0 |
| 1.4 | 0 |
| 1.3 | 0 |
| 1.2 | 0 |
| | |
| unbehandelte, infizierte Kontrolle | 100 |

### Beispiel 5

Ca. 14 Tage alte Ackerbohnen der Sorten "Herz Freya" oder "Frank's Ackerperle" wurden mit wäßrigen Suspensionen der beanspruchten Verbindungen tropfnaß behandelt.

Nach Antrocknen des Spritzbelages wurden die Pflanzen mit einer Sporensuspension (1,5 Mio. Sporen/ml) von Botrytis cinerea inokuliert. Die Pflanzen wurden in einer Klimakammer bei 20-22 °C und ca.99 % rel. Luftfeuchte weiterkultiviert. Die Infektion der Pflanzen äußert sich in der Bildung schwarzer Flecken auf Blättern und Stengeln. Die Auswertung der Versuche erfolgte ca. 1 Woche nach Inokulation.

Der Wirkungsgrad der Prüfsubstanzen wurde prozentual zur unbehandelten infizierten Kontrolle bonitiert und ist in Tabelle 5 wiedergegeben.

**Tabelle 5**

| Verbindung gemäß Beispiel | mit Botrytis cinerea befallene Blattfläche in % bei mg Wirkstoff/Liter Spritzbrühe |
|---|---|
| | 500 |
| 2.1 | 0 |
| unbehandelte, infizierte Pflanzen | 100 |

## Patentansprüche

1. Verbindungen der Formel I 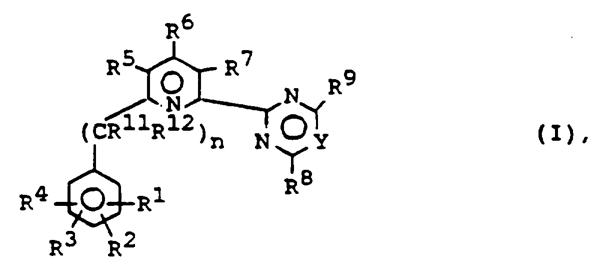 worin
R¹, R, R³, R⁴ = unabhängig voneinander Wasserstoff, Halogen, Nitro, (C₁-C₄)Alkyl, (C₁-C₄)Alkoxy, (C₁-C₄)Alkylthio, (C₁-C₄)Haloalkoxy oder (C₁-C₄)Haloalkyl,
R⁵, R⁶, R⁷ = unabhängig voneinander Wasserstoff, (C₁-C₆)Alkyl oder Phenyl, wobei der Phenylrest bis zu dreifach durch Halogen, Nitro, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
R⁸ und R⁹ = unabhängig voneinander Wasserstoff, (C₁-C₆)Alkyl, (C₃-C₇)Cycloalkyl, (C3-C7)Cycloalkyl-(C₁-C₄)alkyl, wobei die beiden letztgenannten Reste im Cycloalkylteil bis zu dreifach durch (C₁-C₄)Alkyl substituiert sein können, (C₁-C₄)Haloalkyl, (C₁-C₄)Alkoxy, (C₁-C₄)Alkylthio, (C₁-C₄)Alkoxy-(C₁-C₄)alkyl, (C₁-C₄)Alkylthio-(C₁-C₄)alkyl, Halogen, (C₂-C₆)Alkenyl, (C₂-C₆)Alkinyl, Phenyl, Phenoxy, Phenoxy-(C₁-C₄)alkyl, Phenylmercapto-(C₁-C₄)alkyl, Phenylmercapto, Phenyl-(C₁-C₄)alkoxy oder Phenyl-(C₁-C₄)alkylthio, wobei die sieben letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, oder (C₂-C₄Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio-(C₁-C₄)alkylthio,
R¹⁰ = Wasserstoff, (C₁-C₄)Alkyl, (C₁-C₄)Alkoxy, (C₂-C₆)-Alkenyloxy, (C₂-C₆)Alkinyloxy, (C₁-C₄)Alkylthio, Halogen, Phenyl, wobei der Phenylrest bis zu dreifach durch Halogen, Nitro, Cyano, (C₁-C₄)Alkyl, (C₁-C₄)-Alkoxy, (C₁-C₄)Alkylthio, (C₁-C₄)Haloalkyl oder (C₁-C₄)Haloalkoxy substituiert sein kann, oder
R⁸ und R¹⁰ bilden zusammen eine Polymethylenkette der Formel -(CH₂)ₘ- mit m = 3 - 4,
R¹¹ und R¹ = unabhängig voneinander Wasserstoff oder (C₁-C₄)Alkyl,
Y = N oder -CR¹⁰ und
n = 1-3 bedeuten, sowie deren Säureadditionssalze.

2. Verbindungen der Formel I von Anspruch 1, worin
R¹, R, R³, R⁴ = unabhängig voneinander Wasserstoff, Halogen, (C₁-C₂)Haloalkyl oder (C₁-C₃)Alkyl,
R⁵, R⁶ = unabhängig voneinander Wasserstoff, (C₁-C₃)Alkyl oder Phenyl, wobei der Phenylrest bis zu dreifach durch Halogen oder (C₁-C₄)Alkyl substituiert sein kann,
R⁷ = Wasserstoff,
R⁸ und R⁹ unabhängig voneinander Wasserstoff, (C₁-C₆)Alkyl, (C₃-C₆)Cycloalkyl, (C₅-C₆)Cycloalkyl-(C₁-C₃)alkyl, Halogen, Phenyl, das bis zu dreifach durch Halogen, (C₁-C₄)Alkyl oder (C₁-C₄)Alkoxy substituiert sein kann, oder (C₂-C₄)Alkenyloxy, (C₂-C₄)Alkinyloxy, (C₁-C₄)Haloalkoxy, (C₁-C₄)Alkoxy-(C₁-C₄)alkoxy,
R¹⁰ = Wasserstoff, (C₁-C₄)Alkyl, Halogen, Phenyl, wobei der Phenylrest bis zu dreifach durch Halogen oder (C₁-C₃)Alkyl substituiert sein kann, oder (C₁-C₃)Alkoxy oder
R⁸ und R¹⁰ bilden zusammen eine Polymethylenkette der Formel -(CH₂)ₘ- mit m = 3 - 4,
R¹¹, R¹ = Wasserstoff,
Y = N oder -CR¹⁰
und n = 1 bedeuten, sowie deren Säureadditionssalze.

3. Verfahren zur Herstellung von Verbindungen der Formel I gemäß Anspruch 1 oder 2, dadurch gekennzeichnet, daß man
a) für Verbindungen I mit R⁹ = H, Y = -CR¹⁰ und den restlichen Substituenten in den Bedeutungen der Formel I ein entsprechendes Halopyrimidin I, worin R⁹ = Halogen bedeutet, reduktiv dehalogeniert, oder
b) für Verbindungen I, worin R⁹ für (C₁-C₄)Alkoxy, (C₁-C₄)Alkylthio, Phenoxy, Phenylmercapto, Phenyl-(C₁-C₄)alkoxy, oder Phenyl-(C₁-C₄)alkylthio, wobei die 4 letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, oder (C₂-C₄)Alkenyloxy (C₂-C₄)Alkinyloxy, (C₁-C₄)Haloalkoxy, (C₁-C₄)Alkoxy-(C₁-C₄)alkoxy oder (C₁-C₄)Alkylthio-(C₁-C₄)alkylthio steht und Y = -CR¹⁰ bedeutet, ein entsprechendes Halopyrimdin I, worin R⁹ = Halogen bedeutet, mit einer Verbindung der Formel II
R⁹M (II),
wobei R⁹ die obengenannte Bedeutung besitzt und M für ein Alkalimetall steht, umsetzt oder
c) für Verbindungen der Formel I, worin R⁹ für (C₁-C₆)Alkyl, (C₃-C₇)Cycloalkyl, (C₃-C₇)Cycloalkyl-(C₁-C₄)alkyl, (C₁-C₄)Alkoxy-(C₁-C₄)alkyl, (C₁-C₄)Alkylthio-(C₁-C₄)alkyl oder Phenyl steht, wobei der letztgenannte Rest bis zu dreifach durch (C₁-C₄)Alkyl oder (C₁-C₄)Alkoxy substituiert sein kann, und Y = -CR¹⁰ bedeutet, ein entsprechendes Halopyrimidin I mit R⁹ = Halogen mit einer Grignard-Verbindung III
R⁹ MgX (III),
wobei R⁹ die obengenannte Bedeutung hat und X für Halogen steht, in Gegenwart von Nickel-Phosphin-Komplexen umsetzt, oder
d) für Verbindungen der Formel I, worin R⁸ = R⁹ für (C₁-C₄)Alkyl, Phenyl-(C₁-C₄)alkyl oder Phenyl steht, wobei die beiden letztgenannten Reste im Phenylteil bis zu dreifach durch Halogen, Nitro, Cyano, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, und Y = N bedeutet, ein Amidin-Derviat der Formel IV 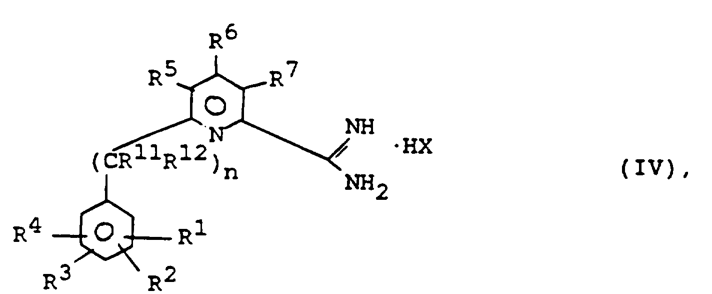 worin R¹-R⁷, R¹¹-R¹ und n die in Formel I angegebene Bedeutung besitzen und X für Halogen steht, mit einem Imidat-Derivat der Formel VI 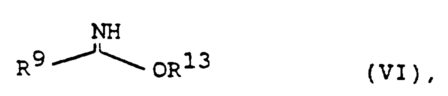 wobei R⁹ die obengenannte Bedeutung hat und R¹³ für (C₁-C₃)Alkyl steht, umsetzt.

4. Fungizide Mittel, dadurch gekennzeichnet, daß sie eine wirksame Menge einer Verbindung der Formel I gemäß Anspruch 1 oder 2 enthalten.

5. Verwendung von Verbindungen der Formel I gemäß Anspruch 1 oder 2 zur Bekämpfung von Schadpilzen.

6. Verfahren zur Bekämpfung von Schadpilzen, dadurch gekennzeichnet, daß man auf die von ihnen befallenen Pflanzen, Flächen oder Substrate eine wirksame Menge einer Verbindung der Formel I gemäß Anspruch 1 oder 2 appliziert.

## Claims

1. A compound of the formula I 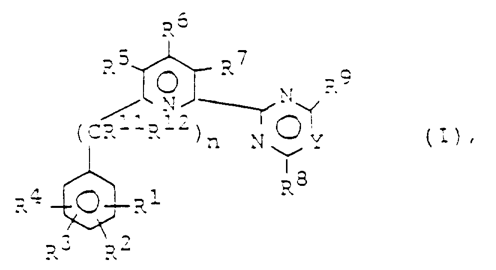 in which
R¹, R, R³ and R⁴ are independently of one another, hydrogen, halogen, nitro, (C₁-C₄)alkyl, (C₁-C₄)alkoxy, (C₁-C₄)alkylthio, (C₁-C₄)haloalkoxy or (C₁ -C₄)haloalkyl,
R⁵, R⁶ and R⁷ are independently of one another hydrogen, (C₁-C₆)alkyl or phenyl, it being possible for the phenyl radical to be monosubstituted to trisubstituted by halogen, nitro, (C₁-C₄)alkyl, (C₁-C₄)alkoxy, (C₁-C₄)alkylthio, (C₁-C₄)haloalkyl, or (C₁-C₄)haloalkoxy,
R⁸ and R⁹ are independently of one another hydrogen, (C₁-C₆)alkyl, (C₃-C₇)cycloalkyl, (C₃-C₇)cycloalkyl-(C₁-C₄)alkyl, it being possible for the two last-mentioned radicals to be monosubstituted to trisubstituted in the cycloalkyl moiety by (C₁-C₄)alkyl, (C₁-C₄)haloalkyl, (C₁-C₄)alkoxy, (C₁-C₄)alkylthio, (C₁-C₄)alkoxy-(C₁-C₄)alkyl, (C₁-C₄)alkylthio-(C₁-C₄)alkyl, halogen, (C₂-C₆)alkenyl, (C₂-C₆)alkynyl, phenyl, phenoxy, phenoxy-(C₁-C₄)alkyl, phenylmercapto-(C₁-C₄)alkyl, phenylmercapto, phenyl-(C₁-C₄)alkoxy or phenyl-(C₁-C₄)alkylthio, it being possible for the seven last-mentioned radicals to be monosubstituted to trisubstituted in the phenyl moiety by halogen, nitro, cyano, (C₁-C₄)alkyl, (C₁-C₄)alkoxy, (C₁-C₄)alkylthio, (C₁-C₄)haloalkyl or (C₁-C₄)haloalkoxy, or (C₂-C₄)alkenyloxy, (C₂-C₄)alkynyloxy, (C₁-C₄)haloalkoxy, (C₁-C₄)alkoxy-(C₁-C₄)alkoxy or (C₁-C₄)alkylthio-(C₁-C₄)alkylthio,
R¹⁰ is hydrogen, (C₁-C₄)alkyl, (C₁-C₄)alkoxy, (C₂-C₆)-alkenyloxy, (C₂-C₆)alkynyloxy, (C₁-C₄)alkylthio, halogen, phenyl, it being possible for the phenyl radical to be monosubstituted to trisubstituted by halogen, nitro, cyano, (C₁-C₄)alkyl, (C₁-C₄)alkoxy, (C₁-C₄)alkylthio, (C₁-C₄)haloalkyl or (C₁₋₄)haloalkoxy, or
R⁸ and R¹⁰ together form a polymethylene chain of the formula -(CH₂)ₘ- where m = 3 - 4,
R¹¹ and R¹ are independently of one another hydrogen or (C₁-C₄)alkyl,
Y is N or -CR¹⁰ and
n is 1-3, and their acid addition salts.

2. A compound of the formula I of claim 1, in which
R¹, R, R³ and R⁴ are independently of one another hydrogen, halogen, (C₁-C₂)haloalkyl or (C₁-C₃)alkyl,
R⁵ and R⁶ are independently of one another hydrogen, (C₁-C₃)alkyl or phenyl, it being possible for the phenyl radical to be monosubstituted to trisubstituted by halogen or (C₁-C₄)alkyl,
R⁷ is hydrogen,
R⁸ and R⁹ are independently of one another hydrogen, (C₁-C₆)alkyl, (C₃-C₆)cycloalkyl, (C₅-C₆)cycloalkyl-(C₁-C₃)alkyl, halogen, phenyl which can be monosubstituted to trisubstituted by halogen, (C₁-C₄)alkyl or (C₁ -C₄)alkoxy, or (C₂-C₄)alkenyloxy, (C₂-C₄)alkynyloxy, (C₁-C₄)haloalkoxy or (C1-C₄)alkoxy-(C₁-C₄)alkoxy,
R¹⁰ is hydrogen, (C₁-C₄)alkyl, halogen, phenyl, it being possible for the phenyl radical to be monosubstituted to trisubstituted by halogen or (C₁-C₃)alkyl, or (C₁-C₃)alkoxy or
R⁸ and R¹⁰ together form a polymethylene chain of the formula -(CH₂)ₘ- where m = 3 - 4,
R¹¹ and R¹ are hydrogen,
Y is N or -CR¹⁰
and n = 1, and their acid addition salts.

3. A process for the preparation of compounds of the formula I as claimed in claim 1 or 2, which comprises
a) for compounds I where R⁹ = H, Y = -CR¹⁰ and the remaining substituents have the meanings of the formula I, reductively dehalogenating an appropriate halopyrimidine I in which R⁹ = halogen, or
b) for compounds I in which R⁹ is (C₁-C₄)alkoxy, (C₁-C₄)alkylthio, phenoxy, phenylmercapto, phenyl-(C₁-C₄)alkoxy or phenyl-(C₁-C₄)alkylthio, it being possible for the 4 last-mentioned radicals in the phenyl moiety to be monosubstituted to trisubstituted by halogen, nitro, cyano, (C₁-C₄)alkyl, (C₁-C₄)alkoxy, (C₁-C₄)alkylthio, (C₁-C₄)haloalkyl or (C₁-C₄)haloalkoxy, or (C₂-C₄)alkenyloxy, (C₂-C₄)alkynyloxy, (C₁-C₄)haloalkoxy, (C₁-C₄)alkoxy-(C₁-C₄)alkoxy or (C₁-C₄)alkylthio-(C₁-C₄)alkylthio and Y is -CR¹⁰, reacting an appropriate halopyrimidine I in which R⁹ = halogen with a compound of the formula II
R⁹M (II),
where R⁹ has the abovementioned meaning and M is an alkali metal, or
c) for compounds of the formula I in which R⁹ is (C₁-C₅)alkyl, (C₃-C₇)cycloalkyl, (C₃-C₇)cycloalkyl-(C₁-C₄)alkyl, (C₁-C₄)alkoxy-(C₁-C₄)alkyl, (C₁-C₄)alkylthio-(C₁-C₄)alkyl or phenyl, it being possible for the last-mentioned radical to be monosubstituted to trisubstituted by (C₁-C₄)alkyl or (C₁-C₄)alkoxy, and Y is -CR¹⁰ reacting an appropriate halopyrimidine I where R⁹ = halogen with a Grignard compound III
R⁹ MgX (III),
where R⁹ has the abovementioned meaning and X is halogen, in the presence of nickel-phosphine complexes, or
d) for compounds of the formula I in which R⁸ = R⁹ and is (C₁-C₄)alkyl, phenyl-(C₁-C₄)alkyl or phenyl, it being possible for the two last-mentioned radicals to be monosubstituted to trisubstituted in the phenyl moiety by halogen, nitro, cyano, (C₁-C₄)alkyl, (C₁-C₄)alkoxy, (C₁-C₄)alkylthio, (C₁-C₄)haloalkyl or (C₁-C₄)haloalkoxy, and Y is N, reacting an amidine derivative of the formula IV 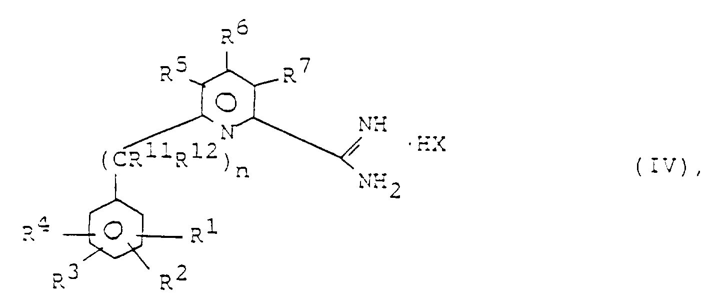 in which R¹-R⁷, R¹¹-R¹ and n have the meaning indicated in formula I and X is halogen, with an imidate derivative of the formula VI 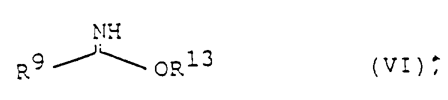 where R⁹ has the abovementioned meaning and R¹³ is (C₁-C₃)alkyl.

4. A fungicidal agent which contains an effective amount of a compound of the formula I as claimed in claim 1 or 2.

5. The use of compounds of the formula I as claimed in claim 1 or 2 for controlling harmful fungi.

6. A method for controlling harmful fungi which comprises applying an effective amount of a compound of the formula I as claimed in claim 1 or 2 to the plants, surfaces or substrates attacked by them.

## Revendications

1. Composés de formule I, 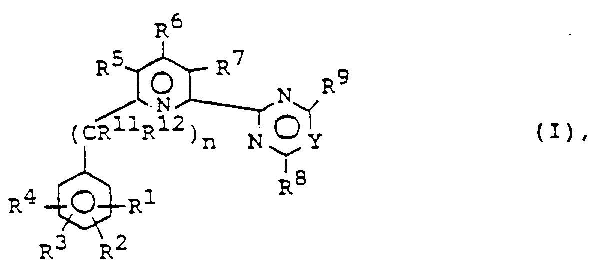 dans laquelle
R¹, R, R³ et R⁴, indépendamment les uns des autres, sont chacun l'hydrogène, un halogène, un groupe nitro, alkyle en C₁-C₄, alcoxy en C₁-C₄, alkylthio en C₁-C₄, halogènalcoxy en C₁-C₄ ou halogènalkyle en C₁-C₄,
R⁵, R⁶, R⁷, indépendamment les uns des autres, sont chacun un hydrogène, un radical alkyle en C₁-C₆ ou phényle, auquel cas le radical phényle peut être jusqu'à trois fois substitué par des substituants halogéno, nitro, alkyle en C₁-C₄, alcoxy en C₁-C₄, alkylthio en C₁-C₄, halogènalkyle en C₁-C₄ ou halogènalcoxy en C₁-C₄,
R⁸ et R⁹, indépendamment l'un de l'autre, sont chacun un hydrogène ou un radical alkyle en C₁-C₆, cycloalkyle en C₃-C₇, (cycloalkyle en C₃-C₇)(alkyle en C₁-C₄), ces deux derniers radicaux pouvant, dans la partie cycloalkyle, être jusqu'à trois fois substitués par des substituants alkyle en C₁-C₄ ; halogènalkyle en C₁-C₄, alcoxy en C₁-C₄, alkylthio en C₁-C₄, (alcoxy en C₁-C₄)(alkyle en C₁-C₄), (alkylthio en C₁-C₄)(alkyle en C₁-C₄), halogéno, alcényle en C2-C6, alcynyle en C₂-C₆, phényle, phénoxy, phénoxy(alkyle en C₁-C₄), phénylmercapto(alkyle en C₁-C₄), phénylmercapto, phényl(alcoxy en C₁-C₄) ou phényl(alkylthio en C₁-C₄), les sept radicaux mentionnés en dernier pouvant dans le fragment phényle être jusqu'à trois fois substitués par des substituants halogéno, nitro, cyano, alkyle en C₁-C₄, alcoxy en C₁-C₄, alkylthio en C₁-C₄, halogènalkyle en C₁-C₄ ou halogènalcoxy en C₁-C₄ ; ou encore alcényloxy en C₂-C₄, alcynyloxy en C₂-C₄, halogènalcoxy en C₁-C₄, (alcoxy en C₁-C₄)(alcoxy en C₁-C₄), (alkylthio en C₁-C₄)(alkylthio en C₁-C₄),
R¹⁰ est un hydrogène, un radical alkyle en C₁-C₄, alcoxy en C₁-C₄, alcényloxy en C2-C6, alcynyloxy en C₂-C₆, alkylthio en C₁-C₄, halogéno, phényle, le radical phényle pouvant être jusqu'à trois fois substitué par des substituants halogéno, nitro, cyano, alkyle en C₁C₄, alcoxy en C₁-C₄, alkylthio en C₁-C₄, halogènalkyle en C₁-C₄ ou halogènalcoxy en C₁-C₄, ou bien
R⁸ et R¹⁰ forment ensemble une chaîne polyméthylène de formule -(CH₂)m-, où m vaut 3-4,
R¹¹ et R¹, indépendamment l'un de l'autre, sont chacun un hydrogène ou un radical alkyle en C₁-C₄,
Y est N ou -CR¹⁰, et
n vaut de 1 à 3, ainsi que leurs sels d'addition avec un acide.

2. Composés de formule I selon la revendication 1, dans lesquels
R¹, R, R³ et R⁴, indépendamment les uns des autres, sont chacun un hydrogène, un radical halogéno, halogènalkyle en C₁-C₂ ou alkyle en C₁-C₃,
R⁵ et R⁶, indépendamment l'un de l'autre, sont chacun un hydrogène, un radical alkyle en C₁-C₃ ou phényle, le radical phényle pouvant être jusqu'à trois fois substitué par des substituants halogéno ou alkyle en C₁-C₄,
R⁷ est un hydrogène,
R⁸ et R⁹, indépendamment l'un de l'autre, sont chacun un hydrogène, un radical alkyle en C₁-C₆, cycloalkyle en C₃-C₆, (cycloalkyle en C₅-C₆)(alkyle en C₁-C₃), halogéno, phényle, qui peut être jusqu'à trois fois substitué par des substituants halogéno, alkyle en C₁-C₄ ou alcoxy en C₁-C₄ ; ou bien alcényloxy en C₂-C₄, alcynyloxy en C₂-C₄, halogènalcoxy en C₁-C₄ ou (alcoxy en C₁-C₄)-(alcoxy en C₁-C₄),
R¹⁰ est un hydrogène, un radical alkyle en C₁-C₄, halogéno, phényle, le radical phényle pouvant être jusqu'à trois fois substitué par des substituants halogéno ou alkyle en C₁-C₃ ; ou encore alcoxy en C₁-C₃, ou bien
R⁸ et R¹⁰ forment ensemble une chaîne polyméthylène de formule -(CH2)m- où m vaut 3-4,
R¹¹ et R¹ sont chacun un hydrogène,
X est N ou -CR¹⁰,
et n vaut 1, ainsi que leurs sels d'addition avec un acide.

3. Procédé pour préparer des composés de formule I selon la revendication 1 ou 2, caractérisé en ce que
a) pour les composés I, avec R⁹ = H, Y = -CR¹⁰, les autres substituants ayant les significations données pour la formule I, on soumet à une déshalogénation par réduction une halogénopyrimidine I correspondante dans laquelle R⁹ est un halogène, ou bien
b) pour les composés I dans lesquels R⁹ est un radical alcoxy en C₁-C₄, alkylthio en C₁-C₄, phénoxy, phénylmercapto, phényl(alcoxy en C₁-C₄) ou phényl(alkylthio en C₁-C₄), ces quatre derniers radicaux pouvant, dans le fragment phényle, être jusqu'à trois fois substitués par des substituants halogéno, nitro, cyano, alkyle en C₁-C₄, alcoxy en C₁-C₄, alkylthio en C₁-C₄, halogénalkyle en C₁-C₄ ou halogénalcoxy en C₁-C₄, ou encore alcényloxy en C₂-C₄, alcynyloxy en C₂-C₄, halogénalcoxy en C₁-C₄, (alcoxy en C₁-C₄)(alcoxy en C₁-C₄) ou (alkylthio en C₁-C₄)(alkylthio en C₁-C₄), et Y est -CR¹⁰, on fait réagir une halogénopyrimidine I correspondante dans laquelle R⁹ est un halogène, avec un composé de formule II
R⁹M (II)
dans laquelle R⁹ a la signification donnée ci-dessus et M est un métal alcalin, ou bien
c) pour les composés de formule I dans laquelle R⁹ est un radical alkyle en C₁-C₆, cycloalkyle en C₃-C₇, (cycloalkyle en C₃-C₇)(alkyle en C₁-C₄), (alcoxy en C₁-C₄)(alkyle en C₁-C₄), (alkylthio en C₁-C₄)(alkyle en C₁-C₄) ou phényle, ce dernier radical pouvant être jusqu'à trois fois substitué par des substituants alkyle en C₁-C₄ ou alcoxy en C₁-C₄ et Y est -CR¹⁰, on fait réagir une halogénopyrimidine correspondante I dans laquelle R⁹ est un halogène avec un composé de Grignard III
R⁹MgX (III)
dans laquelle R⁹ a la signification donnée ci-dessus, et X est un halogène, en présence de complexes nickel-phosphine, ou bien
d) pour les composés de formule I dans laquelle R⁸ = R⁹ et représente un radical alkyle en C₁-C₄, phényl(alkyle en C₁-C₄) ou phényle, ces deux derniers radicaux pouvant, dans le fragment phényle, être jusqu'à trois fois substitués par des substituants halogéno, nitro, cyano, alkyle en C₁-C₄, alcoxy en C₁-C₄, alkylthio en C₁-C₄, halogénalkyle en C₁-C₄ ou halogénalcoxy en C₁-C₄, et Y est N, on fait réagir un dérivé de l'amidine de formule IV 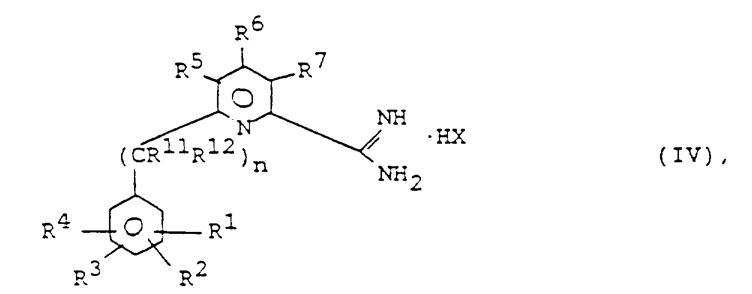 dans laquelle R¹-R⁷, R¹¹-R¹ et n ont les significations données dans la formule I, et X est un halogène, avec un dérivé imidate de formule VI 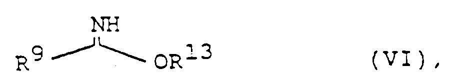 dans laquelle R⁹ a la signification donnée ci-dessus, et R¹³ est un radical alkyle en C₁-C₃.

4. Produits fongicides, caractérisés en ce qu'ils contiennent une quantité efficace d'un composé de formule I selon la revendication 1 ou 2.

5. Utilisation de composés de formule I selon la revendication 1 ou 2 pour maîtriser les champignons nocifs.

6. Procédé pour maîtriser les champignons nocifs, caractérisé en ce qu'on applique sur les plants, surfaces ou substrats infestés par ces derniers, une quantité efficace d'un composé de formule I selon la revendication 1 ou 2.
